# EUROPEAN PATENT APPLICATION

(11) **EP 2 105 155 A1**
(43) Date of publication of application: **30.09.2009**
(21) Application number: 08103053.8
(22) Date of filing: 28.03.2008
(51) Int. Cl.: A61M 5/32, A61M 5/20

(54) **Hollow needle**

(71) Applicant: Hertzog Bernard, 64 Knightsbridge SW1X 7JF (GB)
(72) Inventor: Hertzog Bernard, 64 Knightsbridge SW1X 7JF (GB)
(74) Representative: Perry, Robert Edward

(57) **Abstract**

The present invention is a hollow needle for attachment to a syringe, through which a liquid can be delivered to the *stratum granulosum* during a cosmetic procedure, the needle comprising a distal tip and including a lateral aperture adjacent to the distal tip, wherein the liquid exits the needle via the lateral aperture, and wherein the needle is flexible. Apparatus comprising a needle of the invention and a syringe, and a method of use thereof, is also provided.

## Description

### Field of the Invention

The present invention relates to needles for delivering a liquid to the *stratum granulosum* during a cosmetic procedure.

### Background of the Invention

Many cosmetic procedures carried out on the skin involve the injection of a liquid beneath the epidermis. An example of such a liquid is hyaluronic acid. This is usually injected into the *stratum granulosum*. For injection into the *stratum granulosum*, the needle must pass through the *stratum papillaire*. The *stratum papillaire* contains a network of blood vessels that, if severed, may lead to facial bruising.

Injections of a liquid into the *stratum granulosum* during a cosmetic procedure are traditionally carried out using a conventional sharp pointed needle. One example of such a needle is a 25G needle, which has a diameter of 0.5 mm. Due to its sharp tip, the depth of penetration is difficult to control, and the needle can cut the deep-lying tissues of the skin. In particular, the needle may sever the cellular tissues, the elastic and collagen fibers, and the fine blood and lymph vessels. As the depth of penetration is difficult to control, the administration may not be precise, and the cosmetic procedure may therefore be less effective than desired.

A cosmetic treatment to the skin may require several injections of a liquid. Before each injection with a conventional sharp-tipped needle, anaesthesia is required. This may require a block over a large area.

### Summary of the Invention

The present invention is based on the discovery that, by using a flexible needle with a blunt tip, liquid can be delivered over a large area of the *stratum granulosum*, through one micro-incision. This means that the amount of anaesthesia needed before a cosmetic procedure may be reduced by 10 to 15-fold. As the needle is blunt and flexible, the risk of severing the blood vessels underneath the epidermis may also be reduced.

According to a first aspect, the present invention is a hollow needle for attachment to a syringe, through which a liquid can be delivered to the *stratum granulosum* during a cosmetic procedure, the needle comprising a distal tip and including a lateral aperture adjacent to the distal tip, wherein the liquid exits the needle via the lateral aperture, and wherein the needle is flexible.

According to a second aspect, the present invention is apparatus for delivering a liquid to the *stratum granulosum* during a cosmetic procedure, comprising a syringe and a needle, as defined above.

According to a third aspect, the present invention is a method of delivering a liquid to the *stratum granulosum* during a cosmetic procedure, using apparatus, as defined above.

### Description of Preferred Embodiments

The invention will now be illustrated by way of example only with reference to the following drawings.

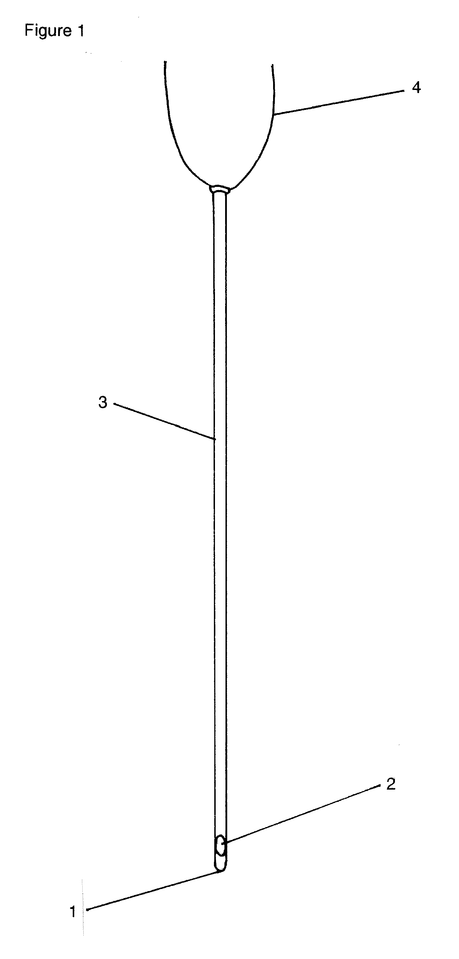

Figure 1 shows a schematic lateral section of a needle embodying the present invention. The needle (3) includes a hollow base section (4), which may be made of a plastics material. This base section (4) allows the needle to be attached to a syringe containing a liquid for subepidermal administration. The needle (3) has a lateral aperture (2), through which the liquid is delivered. The distal tip (1) of the needle is blunt.

The length of a needle according to the invention is preferably between 30 and 70 mm, more preferably between 13 and 45 mm. In a preferred embodiment, the length of the needle is less than 50 mm. In a further preferred embodiment, the length of the needle is approximately 40 mm.

A needle according to the invention preferably has a diameter of between 0.3 and 0.7 mm. More preferably, the diameter is less than 0.9, 0.8, 0.7, 0.6, 0.5, 0.4 or 0.3 mm. More preferably still, the needle has a diameter of approximately 0.5 mm. The fine diameter of the needle may help to reduce the risk of severing the blood vessels as it passes through the mesodermis.

Due to the lateral aperture, the tip of the needle is blunt. Preferably, the tip of needle is rounded. This may reduce the trauma to the patient. The lateral length of the aperture is preferably approximately equal to 0.5 mm. In a preferred embodiment, the aperture is located approximately 1 mm from the distal tip of the needle.

A needle of the invention is flexible, meaning that it may be able to glide through the layers of the skin without damaging the underlying tissues, or cutting the blood vessels. This may help to reduce the amount of bruising after a particular cosmetic procedure.

Due to the flexibility of a needle of the invention, and the resistance offered by the deeper layers of the skin, a liquid can be delivered to the *stratum granulosum* over a large area, and in an even manner, through one single micro-incision of the skin. The tension of the deep-lying layers of skin prevents the blunt needle from penetrating into those layers, and causes it to "curve" into the *stratum granulosum*. The user may be able to manipulate the needle in a "circular" motion, in order to maximize the area of delivery of the liquid. Due to the construction of the needle, it can glide under the skin with ease. This may significantly reduce the pain and trauma to the patient.

Further, as a needle of the invention is able to deliver a liquid to a large area, through one single initial micro-incision, a dosage of anaesthetic to that single micro-incision site may provide sufficient anaesthesia for the injection of all the liquid required for the cosmetic procedure. If a conventional sharp-tipped, inflexible needle were used, several micro-incisions, and several dosages of anaesthetic would normally be required.

By way of example, a cosmetic treatment using conventional sharp-tipped needles may require a dosage of anaesthetic at 20 different sites followed by 20 injections of a liquid such as hyaluronic acid, into each those sites. The same procedure using a needle of the invention may require anaesthetic to be administered to a single site only, followed by a single injection of hyaluronic through one single micro-incision. Due to the construction of the needle, the same area can be treated, as if the procedure was carried out using the conventional method. The requirement for one micro-incision only may greatly reduce the negative side-effects of the cosmetic procedure, such as bruising and scarring. The procedure may also be more precise, leading to more aesthetically pleasing results.

In one embodiment, a kit is provided, comprising a needle of the invention and a syringe. The syringe may contain the liquid medicament, an example of which is hyaluronic acid. Alternatively, the medicament may be provided separately. In another embodiment, the needle of the invention and the syringe may be integral.

The following Example illustrates the invention.

### Example

After disinfecting the skin, a small quantity of anesthetic is injected (10 to 15 times less than the usual amount when using conventional needles). One single micro-incision is then created using a conventional 25G or 26G needle. This micro-incision should be placed in the centre of the area to be treated. The entire length of a needle according to the invention is then inserted laterally into the *stratum granulosum* through this micro-incision, and the desired liquid (for example, reticulated hyaluronic acid) is injected beneath the skin. The injection is then repeated in a radial pattern, resembling the "spokes-of-a-wheel", always starting from the same initial micro-incision. Bruising is greatly reduced compared with a conventional procedure, using sharp-tipped needles.

## Claims

1. A hollow needle for attachment to a syringe, through which a liquid can be delivered to the *stratum granulosum* during a cosmetic procedure, the needle comprising a distal tip and including a lateral aperture adjacent to the distal tip, wherein the liquid exits the needle via the lateral aperture, and wherein the needle is flexible.

2. A needle according to claim 1, wherein the distal tip is rounded.

3. A needle according to claim 1 or claim 2, wherein the diameter of the needle is less than 0.5, 0.4 or 0.3 mm.

4. A needle according to any preceding claim, wherein the length of the needle is between 13 and 45 mm.

5. Apparatus for delivering a liquid to the *stratum granulosum* during a cosmetic procedure, comprising a syringe and a needle according to any of claims 1 to 4.

6. A method of delivering a liquid to the *stratum granulosum* during a cosmetic procedure, using apparatus according to claim 5.

7. A method according to claim 6, wherein the cosmetic procedure is carried out on the face.
